# EUROPEAN PATENT APPLICATION

(11) **EP 4 226 912 A1**
(43) Date of publication of application: **16.08.2023**
(21) Application number: 22155934.7
(22) Date of filing: 09.02.2022
(51) Int. Cl.: A61K 9/00, A61K 9/14, A61K 9/48

(54) **A POLYMER TO INCREASE THE BIOAVAILABILITY OF A PHARMACEUTICAL COMPOUND**

(71) Applicant: CATALYA, 4020 Liège (BE)
(72) Inventor: GRANDFILS, Christian, 4020 Liège (BE); SEVRIN, Chantal, 4020 Liège (BE)
(74) Representative: Calysta NV

(57) **Abstract**

A polymer substantially consisting of a succession of monomers according to the formula I for use in increasing the bioavailability of pharmaceutical component to a mammalian patient, a process for formulating the polymer and the pharmaceutical component.

## Description

### Technical Field

The present invention relates to pharmaceutical component delivery carriers and compositions, methods for making the carriers and compositions, and methods for using the carriers and compositions for pharmaceutical component delivery to a patient or for intracellular delivery.

### Prior art

It is known in the prior art that therapeutic effectiveness of a drug depends upon the bioavailability and ultimately upon the solubility of drug molecules. Solubility is one of the important parameters to achieve a desired concentration of a drug in systemic circulation for a pharmacological response to be shown. It is estimated that 90% in today's drug development pipeline are poorly water soluble.

Beside water solubility, the capacity of a drug to pass through biological barriers (epithelia, nasal mucosa, the digestive tract, the blood-brain barrier, the cell membrane,...) implies constraints on the administration mode, as well as on its efficacity or on its toxicity. For instance, a drug not able to reach its intracellular target or its target in the cerebrospinal fluid is of no value, whereas a drug that cannot be excreted risks an accumulation: of the drug itself or of its metabolites, which is often associated to detrimental toxicity issues.

There are various techniques available to improve the solubility of poorly soluble drugs or the bio-availability of drugs. Some of the approaches to improve the solubility are physical modifications like particle size reduction. Indeed, the solubility of drugs is often intrinsically related to drug particle size. By reducing the article size, the increased surface area improves the dissolution properties of the drug. Some other approaches to improve the solubility are physico-chemical modifications like using solubilizing agents or the grafting of hydrophilic moieties. Indeed, the solubility and/or the bio-availability of drugs can also be improved by various solubilizing materials known as carriers.

A drug carrier is any substrate used in the process of drug delivery which serves to improve the selectivity, effectiveness, and/or safety of drug administration. Drug carriers are primarily used to control the release of a drug into systemic circulation. Drug carriers are used to improve the pharmacokinetic properties, specifically the bioavailability, of many drugs with poor water solubility and/or membrane permeability.

Carriers can be different kind of compounds, from low molecular weight polyethylene glycol (Mw 400) to polymers having a mass of several kDas.

Unfortunately, one limitation behind the use of polymers as carriers to improve the bioavailability of pharmaceutical component are the safety aspects linked to their non degradability for parenteral applications or to detrimental effects associated to the metabolites from such polymers, when degraded in *vivo.* Above a molecular weight of 10 kDa synthetic polymers are normally not eliminable by the kidney, resulting in an accumulation in the body causing potential inflammation and/or deleterious immune reactions and raising critical safety issues.

Another limitation is their lack of diffusion ability: synthetic or natural polymers are normally not able to cross biological barriers such as mucosae : gastro-intestinal barriers, blood brain barriers, nasal barriers.

Document US2010/0150952 discloses protein delivery carriers comprising a polymer conjugate, comprising a pH-responsive polymer and one or more therapeutic agents covalently coupled thereto.

Unfortunately, such methods are not efficient enough or cause additional toxicities issues.

### Brief summary of the invention

It is the object of the invention to provide a pharmaceutical component delivery carriers and compositions, method for making the carriers and compositions, and methods for using the carriers and compositions for intracellular pharmaceutical component delivery.

According to the invention, this object is solved by providing a polymer consisting of a succession of monomers according to the formula I : 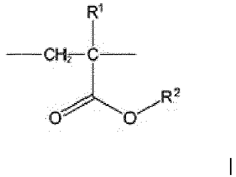 for use in increasing the bioavailability of a pharmaceutical component to a mammalian patient, wherein R¹ represents an hydrogen atom or a straight or branched chain alkyl group from 1 to 6 carbon atoms ; where R² represents a straight or branched chain alkyl group from 1 to 6 carbon atoms, which is substituted by a protonable amino group ; wherein the said polymer has a mean pKa between 6,0 and less than 7,0 and a charge density at pH 7,0 between 20 and 50% (number of the positively charged monomers:total number of monomers).

### Brief description of the Drawings

Figure 1 represents bioavailability and pharmacokinetics study of fluorescein covalently coupled to the polymer of the invention.
Figure 2 represents bioavailability and pharmacokinetics study of fluorescein associated to the polymer of the invention.
Figure 3 represents the polymer of the invention or fluorescein recovery in urine.
Figure 4 represents the polymer of the invention or fluorescein recovery in feces.
Figure 5 represents the absorption spectra of free curcumin and of curcumin associated with the polymer of the invention.

### Detailed description of the invention

The inventors have developed and adapted a polymer to provide properties of increasing the bioavailability of poorly soluble and/or bio-available compounds. Indeed, the inventors have found that the polymer of the invention, even being rather large, can pass through epithelia, and even confer this capacity to a molecule adhered to it. The inventors have further found that the polymer is excreted upon time, which results into a low toxicity profile, a required condition for a therapeutic use.

This allows the administration of a given drug, formulated with the polymer of the present invention, by different modes, including oral or dermal administrations, or by inhalation, or by suppositories.

Moreover, the inventors have found that the cellular uptake of drugs associated with the polymer of the invention is also increased, meaning that drugs, even nucleic acid-based drugs, can be potentiated with the polymer of the present invention. Also drugs with a peptide backbone are also nicely potentiated with the polymer of the present invention.

In the context of the present invention, the word "peptide" preferably refers to an oligomer or a polymer made of a peptidic backbone, with no upper limit. Hence proteins and antibodies or antibody fragments are encompassed. Preferably, the peptidic backbone consists essentially of naturally-occurring amino acids. Modified amino acids, especially the naturally-occurring ones, are also encompassed, such as peptides comprising glycosylated, acylated, hydroxylated or deaminated amino acids.

A first aspect of the present invention is thus a polymer substantially consisting of a succession of monomers according to the formula I 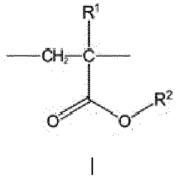
for use in increasing the bioavailability of a pharmaceutical component (adhered to it) to a mammalian patient,
wherein R¹ represents an hydrogen atom or a straight or branched chain alkyl group from 1 to 6 carbon atoms;
wherein R² represents a straight or branched chain alkyl group from 1 to 6 carbon atoms, which is substituted by a protonable amino group

Preferably, this polymer has a mean pKa between 6.0 and less than 7.5, more preferably between 6.5 and 7.0.

Preferably, this polymer has a charge density at pH 7.0 between 20 and 50% (number of the positively charged monomers:total number of monomers).

Preferably, in the context of the present invention, the wording "substantially consisting of a succession of monomers according to the formula I" means that more than 60% , preferably more than 65%, more than 70%, more than 75% or even more than 80% of the monomers are those according to the formula I (monomers according to the formula I: total number of the monomers, including those forming the alpha and omega extremities), preferably more than 85, 90, 91, 92, 93, 94, 95, 96, 97, 98% are those according to the formula I.

The remaining monomers, herein after referred to as "complementary monomers" are preferably of an acrylate structure with no protonable amine, such as methyl methacrylate, ethyl methacrylate, propylmethacrylate or butyl methacrylate.

These complementary monomers will thus be incorporated, possibly in the form of a copolymer with the monomer according to the formula (I).

Examples of copolymeric (and terpolymeric) structures which could be used include poly[2-(dimethylamino)ethyl methacrylate)-co-acrylic acid] (**III**), poly[2-(dimethylamino)ethyl methacrylate)-co-methacrylic acid] (**IV**), poly[2-(dimethylamino) ethyl methacrylate)-co- poly(ethylene glycol) α- methyl ether, co-acrylate] (**V**), poly[2-(dimethylamino) ethyl methacrylate)-co-poly (ethylene glycol) α- methyl ether, co-methacrylate] (**VI**), poly[2-(dimethylamino) ethyl methacrylate)-co- poly(ethylene glycol)] **(**V**II**), poly[2-(dimethylamino) ethyl methacrylate)-co- poly(ethylene glycol)] (**VIII**), poly[methacrylic acid-co-2-(dimethylamino) ethyl methacrylate)-co- poly(ethylene glycol) α-methyl ether, co-methacrylate] (**IX**), poly[methyl methacrylate-co-2-(dimethylamino) ethyl methacrylate)-co- poly(ethylene glycol) α-methyl ether, co-methacrylate] (**X**), poly[trimethylamino) ethyl methacrylate -co-2-(dimethylamino) ethyl methacrylate)-co- poly(ethylene glycol) α-methyl ether, co-methacrylate] (**XI**), poly[trimethylamino) ethyl methacrylate -co-2-(dimethylamino) ethyl methacrylate] (**XII**), poly[2-(dimethylamino) ethyl methacrylate-co-methyl methacrylate-co-butyl methacrylate] of formula (**XIII**); and poly[2-(trimethylamine) ethyl methacrylate chloride-co-ethyl acrylate-co-methyl methacrylate] of formula (**XIV**).

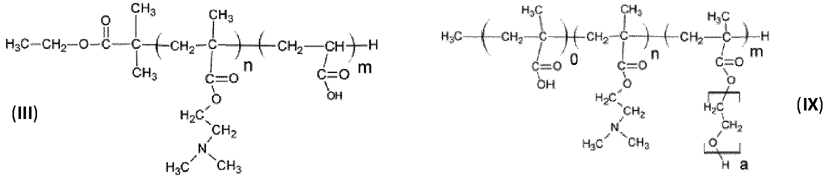

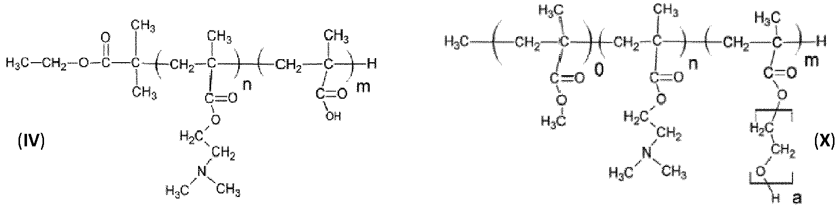

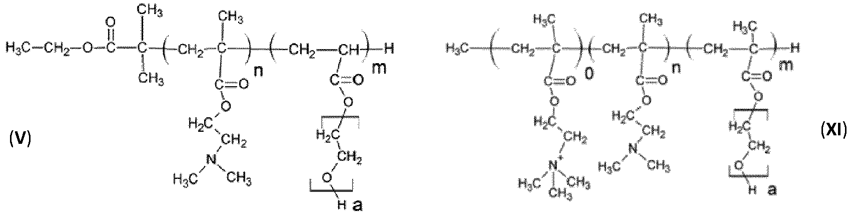

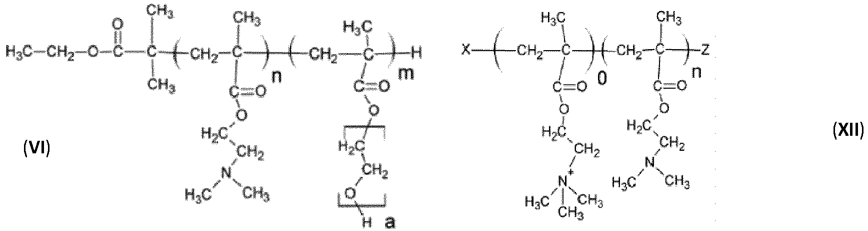

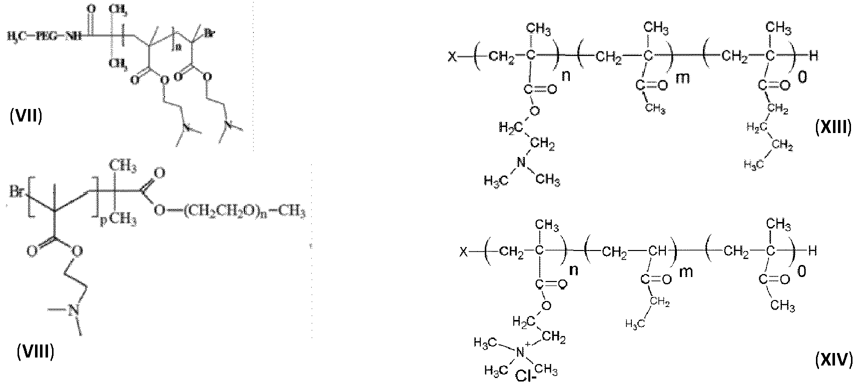

The polymer organization can be adapted depending on the molecule to load: either by random polymerization, or by designing segments consisting of a succession of the monomers according to the Formula I and a succession of the complementary monomers. This allows, for instance, to generate more hydrophobic regions (segments) and more hydrophilic regions (segments) within the same polymer molecule.

The complementary monomers can, possibly, be branched by other oligomeric segments, provided that the polymer keeps its advantageous properties. Possibly, such properties can be screened using an *in vitro* cellular model (CaCo2 cells) and measuring a desired affinity with a drug to be loaded at a pH dependent on the considered administration mode.

The nature, abundance and method of incorporation of the complementary monomers is chosen according to physico-chemical properties needed, essentially the hydrophobicity and net charge of the molecule to load: to a certain extent, more hydrophobic monomers are preferred when the molecule to load is rather hydrophobic, whereas more positively charged monomers are preferred when molecules bearing a heavy net negative charge is to be loaded, such as oligo- or poly-nucleotides.

The selection and architecture of the complementary monomers is, of course, more important when they are incorporated in higher amounts, such as when more than 20 %, more than 25% more than 30% or even close to 40% of the monomers are complementary monomers: a proportion of at least 60% of the monomers corresponding to the Formula I confers the basic structure and physico-chemical properties, and the complementary monomers fine-tune these properties.

The polymerization process can be performed by several methods. Preferably anionic polymerization or free-radical polymerization are used.

Convenient synthesis protocols are indeed based on free-radical polymerization.

ATRP (Atom Transfer Radical Polymerization) is a preferred method of synthesis, especially when there is no need of a specific architecture of the complementary monomers.

RAFT (Reversible Addition Fragmentation Chain Transfer) polymerization has also be successfully found useful for a better control of the polymer organization.

Advantageously, this polymer (where the pharmaceutical compound is adhered or is to be adhered) is administered to the mammalian patient at a daily dose ranging between 1 µg/kg and 300 mg/kg, preferably between 10 µg/kg and 10 mg/kg, more preferably between 100 µg/kg and 1 mg/kg. In other words at least 1, 3, 5, 10, 30, 50 100, 300, 500, 1000, 3000, 5000, 10000, 30000, 50000 or 100000 µg of the polymer is administered by kg of the patient and/or less than 500, 300, 200, 100, 50, 30, 20, 10, 5, 3 and 1 mg of the polymer is administered by kg of the patient.

Preferably, the pharmaceutical component is formulated together with the polymer, preferably as a solid solution or by extrusion at a temperature below 250°C (i.e. between 40°C and 250°C, preferably between 100°C and 230°C), more preferably between 120 and 200°C.

Advantageously, the inventors have found that peptides, including polypeptides, can be processed by extrusion together with the polymer of the present invention, without an irreversible denaturation, despite the increase of the temperature.

In the present invention, preferably, the pharmaceutical compound is poorly soluble in water and/or has a size too big to pass through the cell membranes and/or epithelia by diffusion (e.g. > about 300 Da).

Preferred pharmaceutical compounds are chemotherapeutic agents, antibiotics, terpene compounds, aromatic compounds and/or polyphenols (e.g. curcumin, xanthohumol, catechins, elagitannins and the derivative thereof including urolithin A, B, C or D), peptides or nucleic acids (i.e. oligonucleotides of more than 20 nucleotides, single-stranded or doublestranded; RNA or DNA backbone; natural or modified on the phosphodiester link, on the sugar or the base itself), polynucleotides.

The oligonucleotides can be used for antisense or RNA interference purpose, as well as for an aptamer function. Indeed, the observation of the capacity of the polymer of the present invention to load oligonucleotides and to boost their cytosolic release opens new therapeutic options, either for the direct and specific downregulation of a target RNA, or for allowing specific interaction with proteins or post-translationally-modified peptides, including intracellular proteins, or with other biological moieties (e.g. glycolipids, bacterial or viral structures).

Longer nucleotide molecules (DNA or RNA) are advantageously formulated with the polymer of the present invention for vaccination purpose or for gene therapy.

More into details the peptides to be loaded to the polymers of the present invention can range from a size of more than 10 amino acids to proteins, including antibodies and the fragments thereof, enzymes used in replacement therapy, such as for the treatment of Gaucher (Imiglucerase, Taligucerase alfa), Fabry (agalsidase alfa or beta) or Pompe disease (algucosidase alpha), as well as the coagulation Factor VIII and insulin.

The antibodies or the fragments can be designed to agonize or antagonize a given pathway, by specific fixation on a given target, or can be loaded with a further active moiety, such as a toxic element: the specific adhesion of the antibody will specifically bring the toxic element to the targeted cell. Several antibodies are used or in development, clinical or for the treatment of cancer or inflammatory diseases or even for neurodegenerative diseases. Among them are anti-cytokines (TNF-alpha, IFN-gamma, IL-1, IL-2, IL-4, IgGE, PD-L1, PDGF, VEGF), antibodies targeting amyloid betapeptides (to treat Alzheimer's disease), antibodies fixing to receptor kinase or to kinase (exposed at the cell membrane, or even cytosolic kinases) (e.g. in cancer): anti EGFR, anti HER2, anti CTLA4, Anti PD1, anti CD20, anti VEGFR, anti PDGFR.

More generally drugs for treatment of osteoporosis, bone metabolism improver, hypnotic sedative, sleep inducing agent, anxiolytic, antiepileptic, antidepressant, anti-Parkinson agent, agent for neuropsychiatry, central nervous system agent, local anesthetic, skeletal muscle relaxant, autonomic agent, antipyretic, analgesic, anti-inflammatory agent, antispasmodic agent, vertiginous agent, cardiotonic, antiarrhythmic agent, diuretic, antihypertensive agent, vasoconstrictor, vasodilation, agent for circulatory organs, agent for hyperlipidemia, respiratory stimulant, antitussive, antitussive expectorant, bronchodilator, intestinal agent, peptic ulcer agent, stomach digestive agent, antacid, laxative, gastrointestinal agent, adrenal hormone preparation, hormonal agent, vitamin, hemostatic agent, liver disease agent, diabetic agent, antihistamine agent, antibiotic, antibacterial agent, anti-mosquito agent, anthelminthic agent, antimalarial agent, and/or antineoplastic agent.

Among them, molecules currently locally administered to the cerebrospinal fluid are preferred, including anesthetics, such as ziconotide, which can advantageously be administered by a less invasive action, such as blood injection or ingestion, when formulated with the polymer of the present invention. Nasal administration of molecules designed to be transported to the cerebrospinal fluid is preferred.

More into details, agents such as ticagrelor, ivermectin^{®}, curcumin^{®}, 5-aminoosalicylic acid, acaclovir, adinazolam, aspirin^{®}, acetylsalicylic acid, acasol Minophene, Acetobutol, Acetohexamide, Atenvorrost, Atorvastatin^{®}, Apomorphine, Aminopyrine, Aminophylline^{®}, Ethylaminobenzoate, Amrinone, Amobarbium Oxide, Albuterol^{®}, Alprazolam^{®}, Valopurinol, Ampicillin^{®}, Ambroxol^{®}, Etenzamido, ethosuximide, Etomidorin, ephedrine^{®}, erythromycin^{®}, O alkoxy tetracycline, Kosifurazone, omeprazole^{®}, carmofur, quinidine^{®}, quinine^{®}, griseofulvin^{®}, glipizide^{®}, glucagon^{®}, dalbenclamide, chloramphenicol^{®}, chlordiazepoxide, chlorothiazide^{®}, ketoconazole^{®}, colestimide, codin^{®}, cobamamide, colchicine^{®}, zafirukast, Diazepam^{®}, diquitoxin, diclofenac^{®}, diclofenac sodium^{®}, cyclophosphamide^{®}, digoxin^{®}, cicotiamine, dipyridamole, cimetidine^{®}, josamycin^{®}, simvadin^{®}, scalfarat, scopolamine^{®}, spironolactone^{®}, sulpiride^{®}, sulfasalazine, sulfadimethoxine, sulfamethizone Sulfaguanidine, sulfamethoxazole, sulfur Soxazol, cefotetan, cefuroxime^{®}, Itraconazole^{®}, selegiline^{®}, celecoxib^{®}, tasosartan, tiotepa^{®}, theophylline^{®}, dextromethorphan^{®}, tetracycline^{®}, teprenone, terfenadine, terbium phosphorus, doxorubicin^{®}, tramadol etidrol, triamcinolone^{®}, triamteren, Fenofibrate^{®}, tlutamide, nateglinide, Naproxen^{®}, nicotinic acid amide, nitroglycerin^{®}, nitrofurantoin^{®}, diphadipine, nomonapride, noscapine, hydocorticoid, pafenorol, valdecoxib, valproate^{®}, haloperidol^{®}, hydrocortithine, hydrocortisone^{®}, atovaquone^{®}, Faropenem sodium, Famotidine^{®}, Phenacetin, Phenytoin^{®}, Pheny Pancreas, Primodone, Fluorouracil^{®}, Methylprednisolone^{®}, Prednisolone^{®}, Prednisone^{®}, Phytohormone^{®}, Phenidobarbital, Fenoprofen Calcium, Telmisartan^{®}, Pseudoephedrine^{®}, Budesonide^{®}, Phoformoterol Fumarate, Prodynesemide, probenecid^{®}, bromwareril urea, betamethasone^{®}, penicillin, peroxetine, perfenadine, benzyl penicillin^{®}, penus isosin, calcium hospatenate, polythiazide, chlorophyllumine maleate, midazolam^{®}, milnacipran^{®}, doxazosin mesylate, methyldopa^{®}, metoclobulamide, methotrexate^{®}, metoprolo^{®}, mepirisol Morphine, Ciprofloxacine^{®}, ranitidine^{®}, lansoprazole^{®}, ricinovir, risperidone^{®}, lisoflavin, lidocaine^{®}, codin phosphate, dimorphorphan phosphate, pyridoxal phosphate, reponorussone monel, reserpine^{®}, repodopa, orobaspin, lorazepam^{®}, orobaspin, warfarin^{®}, aclarubicin hydrochloride, Amitriptyline hydrochloride, amosulalol hydrochloride, ampicillin phthalidyl hydrochloride, indenoloyl hydrochloride, ethamptol hydrochloride, indansetron hydrochloride, daranisetron hydrochloride, dulanisetron hydrochloride, chlorpromazine hydrochloride, diphenhydramine hydrochloride, dibecephine hydrochloride, dibein hydrochloride, hydrochloric acid evening mouth, tiapride hydrochloride, terazosin hydrochloride , Bicardipine hydrochloride, barnidipine hydrochloride, hydralazine hydrochloride, bifuemerane hydrochloride, prazosin hydrochloride, hydrochloric acid Pafenon, hydrochloric Moperone, ranitidine hydrochloride, hydrochloric Ramose Tron, scopolamine butylbromide, isosorbide nitrate^{®}, quinidine nitrate, thiamine mononitrate, and/or chloral hydrate are useful.

Preferably, the pharmaceutical compound is adhered to the polymer, meaning, preferably, that under physiological conditions (pH around 7.4, such as between 6.0 and 7.8; 37°C, or even, in case of oral administration with no specific protection, a gastric pH of about 2, whereas a pH of about 6 is usually found in subcellular compartments, such as endosomes), the polymer non-covalently interacts with the pharmaceutical compound, for instance by hydrophobic interactions, ionic interactions, hydrogen bridging, and/or dipole-dipole interactions.

Advantageously, when the formulation is done by extrusion, a pharmaceutically-acceptable adjuvant molecule is added, or can be added if needed, to the polymer in a preliminary step, so as to reduce its Tg temperature (glass transition temperature of the polymer).

Preferred plasticizers are selected from the group consisting of
- bio-based plasticizers, such as Alkyl citrates (e.g., Acetyl triethyl citrate (ATEC), Triethyl citrate (TEC)), triacetin (TA), Methyl ricinoleate,
- epoxidized vegetable oils or castor oil, Vitamin E TPGS (D-α-tocopheryl polyethylene glycol 1000 succinate),
- fatty acid esters (butyl stearate, glycerol monostearate, stearyl alcohol), pressurized carbon dioxide, surfactant (polysorbate 80)
- polyethylene glycol (PEG) or a PEG compound such as, but not limited to, maleimido monomethoxy PEG, activated PEG polypropylene glycol, methoxypoly(ethyleneglycol) polymer. PEG or PEG compounds in the context of the invention can be linear or branched. PEG or PEG compounds in the context of the invention can have a size of about 200Da to about 50 kDa, preferably about 500 Da to about 40 kDa even preferably about 1 kDa to about 20 kDa, such as about 1, 2, 5, 10, 15 or 20 kDa.

Alternatively, the plasticizer can be a di-block copolymer, or multiblock copolymer, described as they contain enough PEG moiety to act as well as a plasticizer.

In other words, preferred plasticizers are polyethylene glycol, citrate esters (triethyl citrate, acetyl tributyl citrate, acetyl triethyl citrate, tributyl citrate), diacetylated monoglycerides, Castor oil, or triacetin.

The plasticizer can be mixed with the polymer of the present invention and the drug (powder) before extrusion. Alternatively, the drug (powder) is mixed firstly with the polymer of the present invention, then the plasticizer is blend to the binary mixture before extrusion.

Advantageously, the polymer (with the loaded drug) is administered by ingestion, by inhalation, by injection, topically (including by a gel), or as suppositories.

Administration by inhalation (nasal administration) has been found very advantageous, especially for vaccines (peptides or nucleic acids), since this mode of administration elicits a more robust cellular immune response, which is important in the response towards viral diseases.

Indeed, the inventors have firstly found that an hydrophobic molecule adhered to the polymer of the invention can pass the epithelia and be excreted, which means that such a molecule, if injected, will be cleared upon time; hence allowing to establish a favorable safety profile for such a molecule. Moreover, several drugs are inactive because they fail to reach their target, for instance drugs that cannot be assimilated upon ingestion or drugs that cannot pass the blood brain barrier, or drugs that need to reach an intracellular target are sometimes difficult to use. The present invention solves this issue in allowing drugs to pass through epithelia and even the blood brain barrier. Moreover, the inventors have found that the polymer of the present invention increases the intracellular delivery of drugs that normally do not enter into the cytosol of a targeted cell. Furthermore, this molecule can be administered to a patient through other modes, less invasive, including by oral administration, since the molecule adhered to the polymer of the invention can pass the intestinal mucosa. Similarly, administration by inhalation (nasal administration) can be considered when this mode is advantageous (e.g. for vaccination or for reaching the cerebrospinal fluid), as well as transdermal administration, where the skin can be permeated by the polymer, or an administration by suppositories. This allows to choose the most convenient mode of administration for the patient's comfort and for minimizing the side-effects and/or maximizing the efficacy of the drug. Moreover, these less-invasive administration modes allow an application on a large-scale, which is particularly useful for drugs needed to be chronically administered or for large-scale vaccination, such as in pandemic situations; furthermore, the polymer of the present invention, when combined with a drug previously known to be administered by an invasive mode, or even by injection (intramuscular, intravenous, intraperitoneal,...), allows to reduce the burden on the medical staff, as well as the costs for the patient and the society, since simpler administration modes can now be chosen. Simpler administration modes furthermore improve the patient's compliance, which is an important issue for drugs needed to be chronically administered.

When the administration mode is ingestion, the polymer and the (non-covalently adhered) pharmaceutical component are preferably encapsulated in a stomach-protecting layer and/or the polymer and the pharmaceutical component are administered to the patient with an empty stomach.

Indeed, the high gastric acidity risks to protonate too heavily the polymer of the invention, which will impair its capacity to interact with hydrophobic molecules and/or will decrease the hydrophobic interactions and even some electrostatic interactions between the polymer of the invention and the pharmaceutical component. One way to counter this is to administer the complex in conditions where the stomachal acidity is reduced; i.e. with no food therein. Another approach, which can be complementary, is to protect the polymer, for instance by using coated-capsules designed for stomachal protection, such as shellac-coated capsules.

Preferably, the weight ratio between the polymer and the pharmaceutical component is ranging between 99.9:0.1 and 50:50 (weight polymer:weight pharmaceutical component), preferably between 90:10 and 60:40, more preferably between 80:20 and 70:30. This allows a correct loading of the pharmaceutical compound on the polymer, and also to deliver enough of the pharmaceutical compound to the patient. When a plasticizer is added (see above), the abundance of the pharmaceutical compound can be slightly reduced, for instance the plasticizer is adhered to half of the loading capacity of the polymer, letting a somehow reduced capacity for the pharmaceutical component (e.g. the pharmaceutical component is present at 10 wt%, to 25 wt%: weight of the pharmaceutical component: sum of the weight of the polymer, the plasticizer and the pharmaceutical component).

In other words at least 1, 3, 5, 10, 30, 50 100, 300, 500, 1000, 3000, 5000, 10000, 30000, 50000 or 100000 µg of the polymer is administered by kg of the patient and/or less than 500, 300, 200, 100, 50, 30, 20, 10, 5, 3 and 1 mg of the polymer is (orally) administered by kg of the patient and, for these values, the weight ratio between the polymer and the pharmaceutical component is ranging between 99.9:0.1 and 50:50 (weight polymer:weight pharmaceutical component), preferably between 90:10 and 60:40, more preferably between 80:20 and 70:30. Suitable values are for instance of about 100, 200 or 300 mg of the polymer/kg of the patient and a weight ratio weight polymer:weight pharmaceutical component) between 70:30 (or 80:20) and 50:50.

For nasal administration, the above doses can be ten-fold lower (from 0.1 µg to 50 mg of the polymer per kg), but the above polymer:active drug ratio is kept.

Another aspect of the present invention is thus a nasal spray device comprising the polymer of the present invention and preferably a peptide or a nucleic acid molecule, possibly with a vaccination adjuvant.

Advantageously, as above-mentioned, the pharmaceutical component is a nucleic acid molecule (comprising at least 10 nucleotides), such an oligonucleotide, dsRNA, a mRNA (vaccine) or a plasmid. The inventors have found a marked increase of the internalization by a target cell of the nucleic acid molecule, when formulated with the polymer of the present invention. Moreover, the inventors have found that the porosity of the endocytic vesicles is increased (the inventors have measured this in function of the capacity of the polymer to be more protonated at acidic pH), allowing a better cytosolic release of the formulated nucleic acid molecule.

Advantageously, the polymer can be loaded with pharmaceutical component having a molecular weight above 290, 300, 350, 500 or even above 1000 Da.

More generally, the polymer to be loaded with the pharmaceutical component has a molecular weight ranging between 1 kDa and 100 kDa, preferably between 5 and 50 kDa, more preferably between 10 and 40 kDa or between 10 and 30 kDa.

Preferably more than 80%, more than 90% and more preferably more than 95% of the monomers forming the polymer are according to the formula I, such as between 95% and 99% (number of the monomers according to the formula I:total number of the monomers).

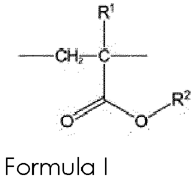

Advantageously, the polymer of the present invention has a charge density at pH 7,0 between 25 and 40%, preferably between 30 and 35% (number of the positively charged monomers:total number of monomers).

Preferably, the polymer of the present invention is bearing less than 10% of negative charge density at pH 7,0, preferably less than 5%, more preferably less than 2% (number of the negatively charged monomers:total number of monomers).

In other words, the polymer of the present invention is slightly charged at pH 7 and/or slightly cationic.

Preferably, one or several of the monomers according to the Formula I and forming the polymer have a R¹ being a methyl group.

Preferably (or in addition), the polymer of the present invention is essentially according to the formula II 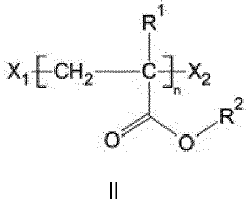
wherein X₁ and X₂ represent the alpha and omega end groups of the said polymer;
wherein the said X₁ and X₂ independently represent a hydrogen atom, a hydroxyl group, an ethyl isobutyrate group, an alkyl group, an halogen group, a carboxylic acid group, an amino group, a methoxy group or an ethoxy group and wherein n is the number of the repetitive monomer units according to the formula I.

Advantageously, as mentioned here above, the increase of bioavailability is the increase of epithelial permeability, preferably the increase of permeability of the intestinal epithelium or of the pulmonary epithelium.

A related aspect of the present invention is process to increase the solubility and/or the bioavailability of a molecule comprising the steps of :
- selecting a molecule in the form of a powder;
- mixing the said powder with a polymer substantially consisting of a succession of monomers according to the formula I 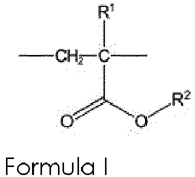
   wherein R¹ represents an hydrogen atom ora straight or branched chain alkyl group from 1 to 6 carbon atoms;
   wherein R² represents a straight or branched chain alkyl group from 1 to 6 carbon atoms, which is substituted by a protonable amino group;
- heating the said mixture at a temperature compatible with the structure and/or function of the said molecule and at a temperature allowing the said polymer to melt;
- extruding the said heated mixture;
- recovering the extruded mixture;
- and optionally formulating the recovered mixture.

Preferably, the polymer used in this process has a mean pKa between 6.00 and 7.5 and/or a charge density at pH 7.0 between 20 and 50% (number of the positively charged monomers:total number of monomers).

This process, as compared to mixtures involving solvents, is simpler, faster (only a few minutes of blending are needed) and safer, especially when toxic solvents are needed in the process of formulating hydrophobic molecules.

The inventors have found that the obtained mixture is very homogenous. Hence a related aspect of the present invention is the molecule formulated according to the above-process, as well as its uses in therapy as described here above and its advantageous administration modes as described here above (nasal and/or inhalation, dermal, oral or as suppositories).

Other characteristics and advantages of the present invention will be derived from the non-limitative following description, and by making reference to the drawings and the examples.

### Example 1 - toxicity

Young adult rats, all of the same category, were included in the study to ensure that 36 (toxicity testing) + 1 (pilot study) animals were available on the first day of treatment. Finally, 34 rats were used for this study. Only females were used in this study because literature surveys of conventional LD50 tests show that, although there is little difference in sensitivity between the sexes, in those cases where differences are observed females are generally slightly more sensitive.

### Pilot study

One animal received by gavage a single oral dose of 300 mg / kg body weight of the polymer of the invention covalently linked to fluorescein.

### Acute toxicity study

The polymers with sizes of 10K (kDa), 25K and 90K were administrated orally by gavage to a group of three experimental animals at one of the defined fixed doses using a stepwise procedure according to Guideline OECD. An interval of minimum 4 days was respected between two steps. For animal welfare, the starting recommended dose was 300 mg / kg bodyweight as there was no preliminary existing information. Administration procedure used in the study is shown in Table 1:

| **Table 1 : Stepwise procedure applied in the present study** | | | | |
|---|---|---|---|---|
| **Group** | **Test item** | **Subgroup** | **Dose** | **Number of rats** |
| A | Catalya 90K (CS104) | A1 | 300 mg/kg | 3 rats |
| | | A2 | | 3 rats |
| | | A3 | 2000 mg/kg | 3 rats |
| | | A4 | | 3 rats |
| B | Catalya 25K (CS107) | B1 | 300 mg/kg | 3 rats |
| | | B2 | | 3 rats |
| | | B3 | 2000 mg/kg | 3 rats |
| | | B4 | | 3 rats |
| C | Catalya 10K (CS103) | C1 | 300 mg/kg | 3 rats |
| | | C2 | | 3 rats |
| | | C3 | 2000 mg/kg | 3 rats |

No mortality was observed at the dose of 300 mg/kg BW. At 2000 mg/kg BW, mortalities were comprised between 0 to 2 / 3 rats (see Table 2) and occurred within 24H after administration.

| **Table 2 : Mortalities in each subgroup** | | | |
|---|---|---|---|
| **Test item** | **Subgroup** | **Dose** | **Dead / TOTAL** |
| Catalya 90K (CS104) | A1 | 300 mg/kg | 0/3 |
| | A2 | | 0/3 |
| | A3 | 2000 mg/kg | 0/3 |
| | A4 | | 2/3 |
| Catalya 25K (CS107) | B1 | 300 mg/kg | 0/3 |
| | B2 | | 0/3 |
| | B3 | 2000 mg/kg | 1/3 |
| | B4 | | 2/3 |
| Catalya 10K (CS103) | C3 | 300 mg/kg | 0/3 |
| | C2 | | 0/3 |
| | C3 | 2000 mg/kg | 2/3 |

The feed intake was stable and comprised between 15 and 24 g per rat at both dose all over the study except on D-1 because rats were fasten and for "Catalya" (i.e. the polymer according to the present invention) 90K 2000 mg/kg BW for which the feed intake remained lower than 15g feed per rat during 3 days after administration).

Signs of toxicity were observed at the dose 2000 mg/kg BW from 2H to 24H after administration and included piloerection, hypothermia, diarrhea, myosis, abnormal posture and gait, tremor, dropping eyelid, dyspnea, muscular hypertonia and behavioural patterns (i.e. increased /decreased reactivity or fear).

### Example 2 - Oral delivery of fluorescein (10 kDa polymer): potentiation by the polymer of the invention

Eighteen rats have been selected and have been given either no polymer (rats 1, 2, 10, 11), or the polymer (10 kDa) at a dose of 5 mg/kg (rats 13-15), 50 mg/kg (rats 4-6, 12, 16-18) or 300 mg/kg (rats 3 and 7-9). Fluorescein has been either covalently coupled to the polymer (rats 4-9 and 13-18; labelled as PO or IV in the figures), or physically (non-covalently) adhered to the polymer (rats 3 and 12); labelled as PO^{∗} or IV^{∗} in the figures.

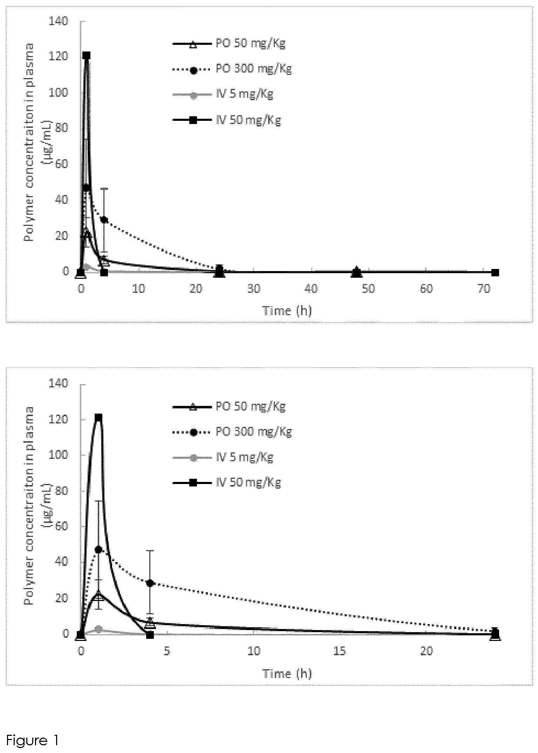

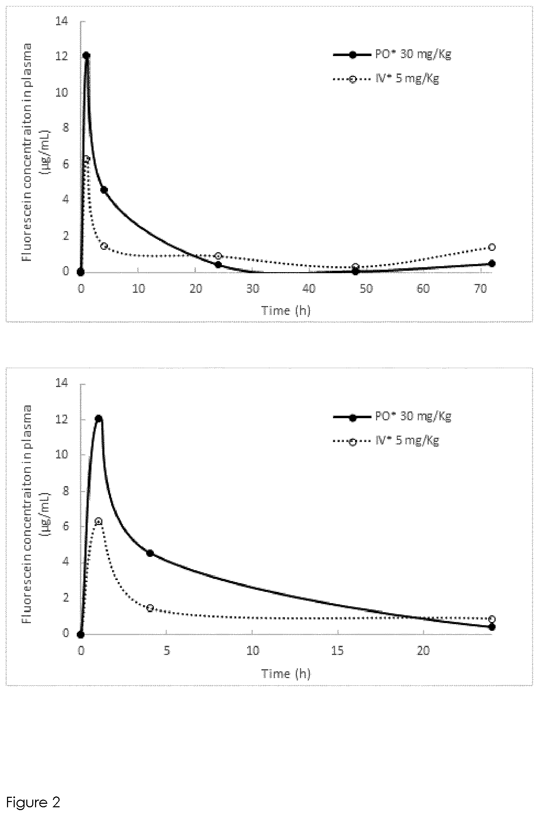

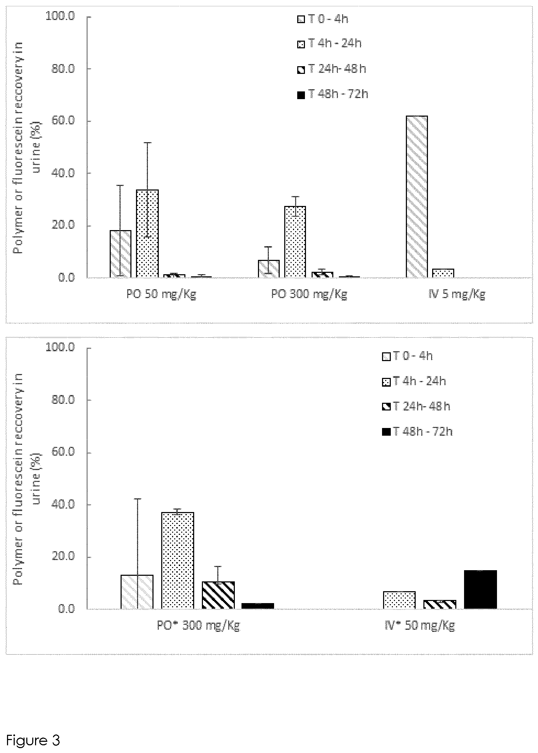

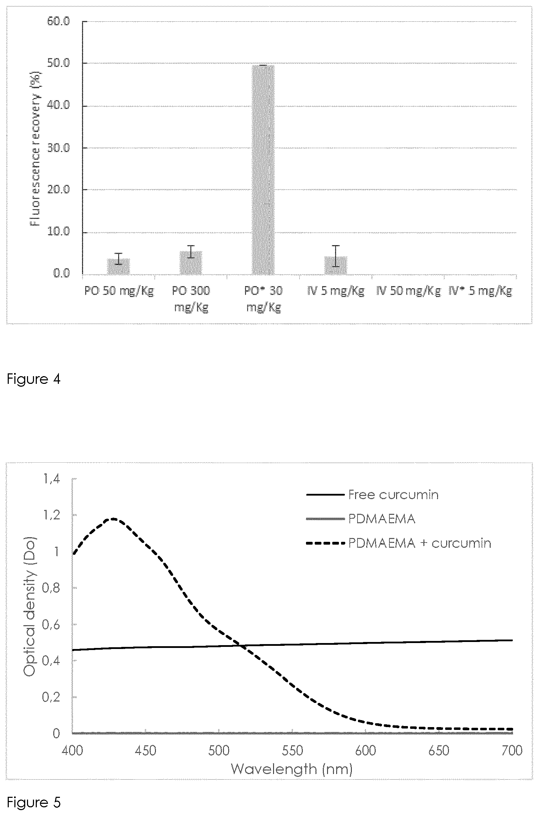

The polymer has been administered either per ingestion (PO or PO^{∗}) or by injection (IV or IV^{∗}). Blood concentration of the polymer-fluorescein (Figure 1), or of the (potentially-free) fluorescein (Figure 2) has been measured in the blood, in the feces (Figure 4) and in urine (Figure 3).

In both situations, significant quantities of fluorescein are measured in the blood, even for ingested compositions, proving that the polymer has been taken up during digestion.

From the measurement on feces samples, it has been estimated that about half of the ingested fluorescein, when non-covalently adhered to the polymer, has been internalized, and then progressively released via urine. Without the polymer, less than 1% of the free fluorescein is internalized, and >99% is found in the feces.

Interestingly, when the fluorescein is non-covalently adhered to the polymer, its blood content is higher, pointing to a slower excretion (evidenced in Figure 3). This means that, when in contact with the blood, the fluorescein is partially detaching from its polymer carrier over time.

On the other hand, the non-covalent association of the polymer and the fluorescein has resisted to a large extent to the stomachal acidity (the composition has been given in absence of food to the animal).

In this experiment, about half of the ingested fluorescein physically (non-covalently) adhered to the polymer of the present invention has been found in the feces (Figure 4), meaning that the other half has been taken up, which is a very good result since no specific stomachal protection has been provided. Conversely, when the fluorescein has been covalently bound to the polymer of the present invention, almost the whole content has been taken up (Figure 4 ; only 3 to 5% in the feces). Together, these two results evidence the usefulness of the polymer of the present invention in allowing a selected molecule to be internalized, but also that care should be taken to protect the non-covalent complex if administered orally.

### Example 3 hot melt blending of curcumin

In this pilot study, 3 grams of the polymer of the present invention (with a formulation being of almost 100% of the monomers according to the Formula I) have been inserted into a mixing room (Plastograph type SEW; blending room W 50 EC) at 140°C, then 1 g of pure curcumin is slowly added upon mixing during 1 minute, before transfer into a separate tube and cooling.

The composition, if dissolved in water, shows an increase of solubility of the curcumin of > 300 times. The 400 nm-700 nm absorption spectrum is also different, with the apparition of a peak at -420 nm (hence a red coloration, vs the normal strong yellow color; Fig. 5).

The inventors have found that the polymers of the present invention can be heated until about 230°C. Hence molecules with a melting point compatible with this temperature, or even a bit higher.

### Example 4 Formulation of Xanthohumol (CAS n° : 6754-58-1)

Xanthohumol has been formulated with a homopolymer of PDMAEMA or with poly[2-(dimethylamino) ethyl methacrylate-co-methyl methacrylate-co-butyl methacrylate with a 10 wt% active loading. The mixing with the different polymers has allowed a solubilization in water of more than 200-fold. After mixing with water, a gel has been formed, with a transition temperature of about 20°C.

It should be understood that the present invention is not limited to the described embodiments and that variations can be applied without going outside of the scope of the claims.

## Claims

1. A polymer substantially consisting of a succession of monomers according to the formula I 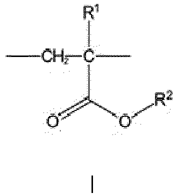
for use in increasing the bioavailability of a pharmaceutical component to a mammalian patient,
wherein R¹ represents an hydrogen atom or a straight or branched chain alkyl group from 1 to 6 carbon atoms;
wherein R² represents a straight or branched chain alkyl group from 1 to 6 carbon atoms, which is substituted by a protonable amino group; wherein the said polymer has a mean pKa between 6.0 and less than 7.5 and a charge density at pH 7.0 between 20 and 50% (number of the positively charged monomers:total number of monomers), wherein the said pharmaceutical component is non-covalently bound and/or adsorbed and/or adhered to the said polymer.

2. The polymer for use in increasing the bioavailability according to claim 1 being administered to the mammalian patient at a daily dose ranging between 1 µg/kg and 300 mg/kg, preferably between 10 µg/kg and 10 mg/kg, more preferably between 100 µg/kg and 1 mg/kg.

3. The polymer for use according to claim 1 or 2, wherein the pharmaceutical component is formulated together with the said polymer, preferably in a solvent-free manner, more preferably as a solid solution or by extrusion at a temperature below 250°C.

4. The polymer for use according to any one of the preceding claims being for administration by ingestion, by inhalation (nasal), by injection, topically (including by a gel or embedded in a dermal patch), or as suppositories, preferably, wherein the administration mode is by ingestion, wherein the said polymer and the pharmaceutical component are encapsulated in a stomach-protecting layer and/or wherein the said polymer and the said pharmaceutical component are administered to the patient with an empty stomach or preferably wherein the administration mode is nasal and wherein the pharmaceutical component is a peptide or a nucleic acid molecule.

5. The polymer for use according to any one of the preceding claims, wherein the weight ratio between the said polymer and the pharmaceutical component is ranging between 99.9:0.1 and 50:50 (weight polymer:weight pharmaceutical component), preferably between 90:10 and 60:40, more preferably between 80:20 and 70:30.

6. The polymer for use according to any one of the preceding claims, wherein the pharmaceutical component has a molecular weight above 290 Da, preferably above 350 DA, preferably above 500 Da, more preferably above 1000 Da, and/or, wherein the said polymer has a molecular weight ranging between 1 kDa and 100 kDa, preferably between 5 and 50 kDa, more preferably between 10 and 30 kDa.

7. The polymer for use according to any one of the preceding claims, wherein the pharmaceutical component is selected from the group consisting of polyphenol derivatives, aromatic compounds, terpene compounds, peptides, nucleic acids, and antibiotics, preferably, wherein the pharmaceutical component is a nucleic acid molecule comprising at least 10 nucleotides.

8. The polymer for use according to any one of the preceding claims comprising more than 80%, preferably more than 90% and more preferably more than 95% of the monomers according to the formula I, such as between 95% and 99% (number of the monomers according to the formula I:total number of the monomers), and/orwherein R¹ is a methyl group.

9. The polymer for use according to any one of the preceding claims having a charge density at pH 7,0 between 25 and 40%, preferably between 30 and 35% (number of the positively charged monomers:total number of monomers), and/or bearing less than 10% of negative charge density at pH 7,0, preferably less than 5%, more preferably less than 2% (number of the negatively charged monomers:total number of monomers).

10. The polymer for use according to any one of the preceding claims, being according to the formula II 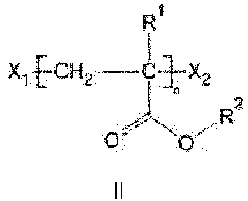
wherein X₁ and X₂ represent the alpha and omega end groups of the said polymer;
wherein the said X₁ and X₂ independently represent a hydrogen atom, a hydroxyl group, an ethyl isobutyrate group, an alkyl group, a halogen group, a carboxylic acid group, an amino group, a methoxy group or an ethoxy group and wherein n is the number of the repetitive monomer units according to the formula I.

11. The polymer for use according to any one of the preceding claims, wherein the increase of bioavailability is the increase of epithelial permeability, preferably the increase of permeability of the nasal epithelium, the intestinal epithelium or of the pulmonary epithelium.

12. A process to increase the solubility and/orthe bioavailability of a molecule comprising the steps of :
- selecting a molecule in the form of a powder;
- mixing the said powder with a polymer substantially consisting of a succession of monomers according to the formula I 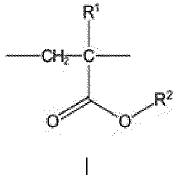
wherein R¹ represents an hydrogen atom or a straight or branched chain alkyl group from 1 to 6 carbon atoms;
wherein R² represents a straight or branched chain alkyl group from 1 to 6 carbon atoms, which is substituted by a protonable amino group;
- heating the said mixture at a temperature compatible with the structure and/or function of the said molecule and at a temperature allowing the said polymer to melt;
- extruding the said heated mixture;
- recovering the extruded mixture;
- and optionally formulating the recovered mixture
preferably, wherein the said polymer has a mean pKa between 6.0 and 7.5 and/or a charge density at pH 7.0 between 20 and 50% (number of the positively charged monomers:total number of monomers).

13. The process of claim 12, wherein the molecule is selected from the group consisting of polyphenol derivatives, aromatic compounds, terpene compounds, peptides, nucleic acids, and antibiotics.

14. The process of claim 12 or 13, comprising a preliminary step of heating a mixture of the polymer with a plasticizer at a temperature allowing the mixed polymer to melt, then of extruding the mixture comprising the said polymer, the said plasticizer and the molecule.

15. A nasal spray device comprising the polymer according to any one of the preceding claims 1 to 11 or the molecule obtained by the process according to any one of the preceding claims 12 to 14, the said nasal spray preferably further comprising a peptide or a nucleic acid molecule, and possibly a vaccination adjuvant.
